(19) Europäisches Patentamt
European Patent Office
Office européen des brevets

(11) Publication number: **0 164 786**
**B1**

(12) # EUROPEAN PATENT SPECIFICATION

(45) Date of publication of patent specification: **14.06.89**

(51) Int. Cl.$^4$: **C 07 C 143/46**

(21) Application number: **85200761.6**

(22) Date of filing: **13.05.85**

(54) **A process for the preparation of p-isononanoyloxybenzenenesulphonate.**

(30) Priority: **08.06.84 GB 8414696**

(43) Date of publication of application:
**18.12.85 Bulletin 85/51**

(45) Publication of the grant of the patent:
**14.06.89 Bulletin 89/24**

(84) Designated Contracting States:
**BE CH DE FR GB IT LI NL SE**

(56) References cited:
**US-A-3 925 016**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Bolsman, Theodorus Antonius B.M.**
**Badhuisweg 3**
**NL-1031 CM Amsterdam (NL)**
Inventor: **van Ginkel, Roelof**
**Badhuisweg 3**
**NL-1031 CM Amsterdam (NL)**
Inventor: **Krijnen, Wilhelmus Johannes**
**Badhuisweg 3**
**NL-1031 CM Amsterdam (NL)**

(74) Representative: **Aalbers, Onno et al**
**P.O. Box 302**
**NL-2501 CH The Hague (NL)**

**EP 0 164 786 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to a process for the preparation of p-isononanoyloxybenzenesulphonate.

p-Isononanoyloxybenzenesulphonate belongs to the group of alkanoyloxybenzenesulphonates, which are disclosed in US patent specifications 4,412,934 as bleach activators in bleaching compositions.

The isononanoyl group of the p-isononanoyloxybenzenesulphonate is derived from the acid 3,5,5-trimethylhexanoic-acid, also called isononanoic acid. The acid has the chemical formula

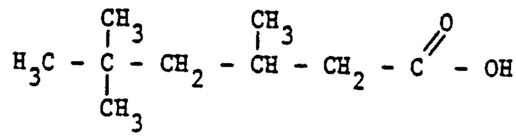

Compounds of general formula

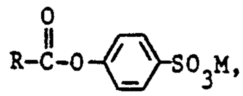

wherein R is an alkyl group and M is sodium or potassium, can be prepared by several processes. German patent publication 2602510 discloses a process wherein sodium p-phenolsulphonate is reacted with acetic anhydride. In US patent specification 3,925,016 is disclosed in Example 1 the preparation of potassium p-oleyloxybenzenesulphonate by reaction of oleic acid chloride with potassium p-phenolsulphonate. However the yield of potassium p-oleyloxybenzenesulphonate is low.

In an attempt to synthesize bleach activators containing an isononanoyl group Applicant found that by reacting isononanoic acid chloride with sodium p-phenolsulphonate in the presence of a non-reactive diluent at a temperature in the range of 140—145°C, an end-product having a sodium p-isononanoyloxybenzenesulphonate content of 84%wt was obtained, a result which could not be increased by prolonged heating and/or addition of further isononanoic acid chloride. In view of the results obtained in the process according to US patent specification 3,925,016, it could not be expected that the corresponding potassium p-isononanoyloxybenzenesulphonate would give a good result.

Surprisingly it has now been found that potassium p-isononanoyloxybenzenesulphonate can be prepared in high yields by a process wherein a high degree of potassium p-phenolsulphonate conversion is achieved. The invention relates to a process for the preparation of p-isononanoyloxybenzenesulphonate characterized in that isononanoic acid chloride is reacted with potassium p-phenolsulphonate in the presence of a non-reactive diluent at a temperature in the range of from 80°C to 200°C. It has also been found that mixtures of potassium and sodium p-phenolsulphonate can be used.

Although the isononanoic acid chloride reacts with the p-phenolsulphonate in an equimolar ratio according to the following reaction scheme:

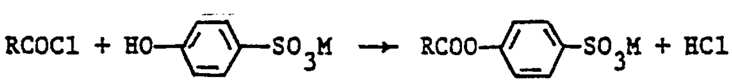

preference is given to a process wherein a slight excess of isononanoic acid chloride over p-phenolsulphonate is employed e.g. a molar ratio in the range of 1.1:1 to 1.5:1. The potassium and/or sodium p-phenolsulphonates are commercially available from numerous suppliers. As sodium p-phenolsulphonate contains 2 moles of water per mol of phenolsulphonate contains 2 moles of water per mol of phenolsulphonate it is essential that the water is removed before use. This can be achieved by drying or azeotropic distillation. When carrying out the reaction with a mixture of potassium and sodium p-phenolsulphonate it is desirable, in order to achieve the improved p-phenolsulphonate conversion, that more than 25% mol of the p-phenolsulphonate is potassium p-phenolsulphonate.

Diluents, which are used in the process according to the invention will not react with any of the reaction components. They can be selected from the aliphatic, cycloaliphatic or aromatic hydrocarbons, although more polar organic diluents such as ethers and ketones may also be employed. Examples of suitable diluents are heptane, isooctane, cyclohexane, n-heptane, benzene, toluene, o-, m- and p-xylene, ethylbenzene, p-methylcumene, mesitylene, monochlorobenzene, anisole, diphenylether, methylisobutylketone, higher boiling aromatic solvents such as SHELLSOL/A (registered trade mark) or mixtures thereof. Aromatic hydrocarbons are preferred. It may be beneficial to employ those non-reactive diluents which are solvents for the isononanoic acid chloride and moreover at ambient temperatures are non-solvents for the p-phenolsulphonates and the p-isononanoyloxybenzenesulphonates. When these conditions are fulfilled it can be expected, upon termination of the reaction and after cooling, that the p-isononanoyloxybenzenesulphonate will precipitate and can be recoverd via filtration together with any non-converted phenolsulphonate and any other insoluble matter which may be present, whereas any isononanoic acid chloride will remain in solution. Optionally depending on the nature of the non-reactive

diluent employed, the precipitation of p-isononanoyloxybenzenesulphonate can be enhanced by the addition of a different non-reactive diluent, which is a weaker solvent for the end-product. Should this still result in an incomplete recovery of the ultimate product, then this can be remedied by including a distillation step before cooling to remove the bulk of the diluent and replace it with a diluent having less solvent power for the end-product. For reasons of processability it was proven to be advantageous to carry out the reaction in the presence of such an amount of non-reactive diluent that the concentration of p-phenolsulphonate is less than 1 mol/l diluent.

The temperature at which the reaction between isononanoic acid chloride and sodium and/or potassium p-phenolsulphonate is carried out is within the range of from 80°C to 200°C. Preferably the reaction is carried out at the reflux temperature of the non-reactive diluent employed since the HCl is removed from the reaction mixture. It is especially preferred to carry out the reaction in refluxing o-, m- or p-xylene or a mixture of two or three of these isomers.

The recovered end-product can be washed at ambient or elevated temperature, employing the same non-reactive diluent as used for the reaction or alternatively the end-product may be washed with another suitable diluent such as pentane, to remove any residual isononanoic acid chloride and after subsequent filtration and drying a solid end-product is obtained which can be used in e.g. laundering compositions.

The invention will be further illustrated by the following examples in which various terms are defined as follows:

SPS: Sodium p-phenolsulphonate. This product had a purity of >99% wt as determined by $^{13}C$ NMR (nuclear magnetic resonance). Prior to use this material was dried at a temperature of 150°C and a pressure of 20 kPa during 18 hours.

KPS/S: Potassium p-phenolsulphonate. 800 ml of Bio-Rad 50 WX2 (registered trademark) (100—200 mesh), containing 0.7 meq H/ml was mixed with 2 l of methanol/water (volume ratio 1/1) and 40 g of SPS. This mixture was stirred at room temperature for 3 hours whereupon the cation-exchange resin was removed by filtration. Subsequently 12 g KOH (as 10% wt in aqueous solution) was added to neutralize the p-phenolsulphonic acid and the neutralized solution was placed overnight on a waterbath to evaporate water and methanol and the obtained residue dried at a temperature of 150°C and a pressure of 20 kPa during 18 hours.

KPS/T: Potassium p-phenolsulphonate, technical grade. This commercially available grade was found to have a potassium p-phenolsulphonate content of approximately 90% wt. Prior to use this material was dried at a temperature of 150°C and a pressure of 20 kPa during 18 hours.

Examples 1—8

Into a three-necked 500 ml glass reactor, equipped with a thermometer, a reflux condenser and a mechanical stirrer made from glass, were introduced predried potassium p-phenolsulphonate or a blend of potassium and sodium p-phenolsulphonate, diluent (o-xylene or toluene) and isononanoic acid chloride in amounts given in the Table. While stirring the obtained mixture was heated to reflux temperature (approximately 140—145°C for o-xylene and 115—120°C for toluene) in about 30 minutes. Heating was effected by placing the glass reactor in an oilbath. At the end of the reaction time the reactor contents were allowed to cool to ambient temperature and subsequently solids were recovered from the reaction mixture by means of filtration. The isolated solids were washed with 50 ml of the diluent used in the preparation and isolated by filtration. This procedure was repeated three times and after the last washing step the solids were dried at a temperature of 100°C and a pressure of 20 kPa for 3 hours. The end-products were analysed to determine the p-isononanoyloxybenzenesulphonate content, employing a two-phase titration according to ISO method 2271 by $^{1}H$ or $^{13}C$ NMR. The results are given in the Table.

Comparative Example A

The comparative example was carried out in the same equipment as used for examples 1—8 and under essentially the same conditions but employing sodium p-phenolsulphonate. The final product was analysed by the above-mentioned techniques and the result is given in the Table.

TABLE

| Example | Isononanoic acid chloride mol | KPS/S mol | KPS/T mol | SPS mol | Diluent | vol. ml | Reaction condition | %wt p-isononanoyl-oxybenzenesulphonate in end-product | Reaction time h |
|---|---|---|---|---|---|---|---|---|---|
| 1 | 0.28 | 0.2 | - | - | o-xylene | 200 | reflux | 97 | 6 |
| 2 | 0.28 | - | 0.2 | - | o-xylene | 200 | reflux | 92 | 6 |
| 3 | 0.28 | - | 0.16 | 0.04 | o-xylene | 200 | reflux | 93 | 6 |
| 4 | 0.28 | - | 0.1 | 0.1 | o-xylene | 200 | reflux | 92 | 6 |
| 5 | 0.28 | - | 0.08 | 0.12 | o-xylene | 200 | reflux | 92 | 6 |
| 6 | 0.4 | 0.2 | - | - | o-xylene | 200 | reflux | 96 | 6 |
| 7 | 0.28 | - | 0.2 | - | o-xylene | 100 | reflux | 90 | 6 |
| 8 | 0.28 | - | 0.2 | - | toluene | 200 | reflux | 88 | 22 |
| A (comparison) | 0.28 | - | - | 0.2 | o-xylene | 200 | reflux | 84 | 24 |

**Claims**

1. A process for the preparation of p-isononanoyloxybenzenesulphonate, characterized in that isononanoic acid chloride is reacted with potassium p-phenolsulphonate in the presence of a non-reactive diluent at a temperature in the range of from 80°C to 200°C.

2. A process according to claim 1, characterized in that part of the potassium p-phenolsulphonate is replaced by sodium p-phenolsulphonate.

3. A process according to claim 1 or 2, characterized in that isononanoic acid chloride is used in a molar excess to the sulphonate(s).

4. A process according to claim 2 or 3, characterized in that the molar amount of potassium p-phenolsulphonate is more than 25% of the total molar amounts of sodium and potassium p-phenolsulphonate.

5. A process according to any one of the claims 1 to 4, characterized in that the molar ratio of isononanoic acid chloride to the sulphonate is in the range of 1.1:1 to 1.5:1.

6. A process according to any one of the claims 1 to 5, characterized in that p-phenolsulphonate is employed in a concentration of less than 1 mol p-phenolsulphonate per litre of non-reactive diluent.

7. A process according to any one of the claims 1 to 6, characterized in that the non-reactive diluent is an aromatic hydrocarbon.

8. A process according to any one of the claims 1 to 7, characterized in that the reaction is carried out at refluxing temperature of the non-reactive diluent.

9. A process according to claim 7, characterized in that the non-reactive diluent is o-, m- or p-xylene or mixtures of these isomers.

**Patentansprüche**

1. Ein Verfahren zur Herstellung von p-Isononanyloxybenzolsulfonat, dadurch gekennzeichnet, daß Isononansäurechlorid mit Kalium-p-phenolsulfonat in Gegenwart eines nicht-reaktiven Verdünnungsmittels bei einer Temperatur im Bereich von 80°C bis 200°C umgesetzt wird.

2. Ein Verfahren nach Anspruch 1, dadurch gekennzeichnet, daß ein Teil des Kalium-p-phenolsulfonats durch Natrium-p-phenolsulfonat ersetzt wird.

3. Ein Verfahren nach Anspruch 1 oder 2, dadurch gekennzeichnet, daß Isononansäurechlorid in einem molaren Überschuß gegenüber dem(den) Sulfonat(en) eingesetzt wird.

4. Ein Verfahren nach Anspruch 2 oder 3, dadurch gekennzeichnet, daß die molare Menge an Kalium-p-phenolsulfonat mehr als 25% der gesamten molaren Menge an Natrium- und Kalium-p-phenolsulfonat ausmacht.

5. Ein Verfahren nach einem der Ansprüche 1 bis 4, dadurch gekennzeichnet, daß das molare Verhaltnis von Isononansäurechlorid zu Sulfonat im Bereich von 1,1:1 bis 1,5:1 liegt.

6. Ein Verfahren nach einem der Ansprüche 1 bis 5, dadurch gekennzeichnet, daß p-Phenolsulfonat in einer Konzentration von weniger als 1 Mol p-Phenolsulfonat pro Liter nicht-reaktiven Verdünnungsmittells eingesetzt wird.

7. Ein Verfahren nach einem der Ansprüche 1 bis 6, dadurch gekennzeichnet, daß das nicht-reaktive Verdünnungsmittel ein aromatischer Kohlenwasserstoff ist.

8. Ein Verfahren nach einem der Ansprüche 1 bis 7, dadurch gekennzeichnet, daß die Reaktion bei Rückflußtemperatur des nicht-reaktiven Verdünnungsmittels durchgeführt wird.

9. Ein Verfahren nach Anspruch 7, dadurch gekennzeichnet, daß das nicht-reaktive Verdünnungsmittel o-, m- oder p-Xylol oder Mischungen dieser Isomere ist.

**Revendications**

1. Procédé de préparation de p-isononanoyloxybenzènesulfonate, caractérisé en ce qu'on fait réagir le chlorure de l'acide isononanoïque avec le p-phénolsulfonate de potassium en présence d'un diluant non réactif à une température située dans un intervalle allant de 80°C à 200°C.

2. Procédé selon la revendication 1, caractérisé en ce qu'on remplace une partie du p-phénolsulfonate de potassium par du p-phénolsulfonate de sodium.

3. Procédé selon la revendication 1 ou 2, caractérisé en ce qu'on utilise du chlorure de l'acide isononanoïque en un excès molaire par rapport au(x) sulfonate(s).

4. Procédé selon la revendication 2 ou 3, caractérisé en ce que la quantité molaire de p-phenolsulfonate de potassium est supérieure à 25% des quantités molaires totales de p-phenolsulfonates de sodium et de potassium.

5. Procédé selon l'une quelconque des revendications 1 à 4, caractérisé en ce que le rapport molaire du chlorure de l'acide isononanoique au sulfonate est dans un intervalle de 1,1:1 à 1,5:1.

6. Procédé selon l'une quelconque des revendications 1 à 5, caractérisé en ce qu'on emploie le p-phénolsulfonate à une concentration inférieure à 1 mole de p-phénolsulfonate par litre de diluant non réactif.

7. Procédé selon l'une quelconque des revendications 1 à 6, caractérisé en ce que le diluant non réactif est une hydrocarbure aromatique.

8. Procédé selon l'une quelconque des revendications 1 à 7, caractérisé en ce qu'on conduit la réaction à la température de reflux du diluant non réactif.

9. Procédé selon la revendication 7, caractérisé en ce que le diluant non réactif est le o-, m- ou p-xylène ou des mélanges de ces isomères.